(19) Europäisches Patentamt / European Patent Office / Office européen des brevets

(11) **EP 4 302 055 B1**

(12) **EUROPEAN PATENT SPECIFICATION**

(45) Date of publication and mention
of the grant of the patent:
**02.07.2025 Bulletin 2025/27**

(21) Application number: **21715690.0**

(22) Date of filing: **05.03.2021**

(51) International Patent Classification (IPC):
**G01H 9/00** *(2006.01)*      **A61B 17/22** *(2006.01)*
**G01D 5/353** *(2006.01)*      **H04B 10/80** *(2013.01)*

(52) Cooperative Patent Classification (CPC):
**G01H 9/004; G01D 5/35354;** A61B 18/26

(86) International application number:
**PCT/SI2021/050009**

(87) International publication number:
**WO 2022/186783 (09.09.2022 Gazette 2022/36)**

(54) **A FAST AND HIGHLY SENSITIVE REFLECTIVE FIBER-OPTIC HYDROPHONE**

SCHNELLES UND HOCHEMPFINDLICHES REFLEKTIERENDES FASEROPTISCHES HYDROPHON

HYDROPHONE À FIBRE OPTIQUE PAR RÉFLEXION RAPIDE ET HAUTEMENT SENSIBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.01.2024 Bulletin 2024/02**

(73) Proprietor: **Univerza V Ljubljani**
**1000 Ljubljana (SI)**

(72) Inventors:
• **PETKOVSEK, Rok**
**1000 Ljubljana (SI)**
• **PETELIN, Jaka**
**1000 Ljubljana (SI)**

(74) Representative: **Patentni Biro AF d.o.o.**
**Kotnikova 32, p.p. 2706**
**1001 Ljubljana (SI)**

(56) References cited:
WO-A1-2019/162664     CN-A- 107 192 439
CN-B- 108 400 516     FR-A1- 2 913 110

## Description

### Field of the invention

The present invention belongs to the field of measuring mechanical vibrations and ultrasonic pressure waves such as those produced by laser induced breakdown in liquids using optical means. The invention is a reflective fiber-optic probe hydrophone with improved bandwidth and sensitivity.

### Background of the invention and the technical problem

Short and high pressure transients in fluids are produced in many industrial and medical applications, for example laser induced breakdown spectroscopy, shock wave lithotripsy and laser vitreolysis. Measuring pressure waves in liquids is usually performed using a hydrophone. Several types of hydrophones already exist, such as those based on a piezoelectric sensor, PDVF membrane or needle hydrophone. The main disadvantages of such hydrophones is a relatively slow response (low bandwidth) and low damage threshold. For measuring shockwaves such as those produced by laser induced breakdown in liquids, a fast (bandwidth above 1 GHz) hydrophone capable of withstanding positive and negative pressures in the range of kbar is required and a fiber-optic hydrophone meets those requirements.

There are two types of fiber-optic hydrophones. The first type uses the principle of interference, where the oncoming pressure waves change the optical path difference between two light beams, producing an interferometric response which can be measured and the amplitude of the pressure waves can then be determined.

The present invention, however, is based on another type of a fiber-optic hydrophone, i.e. a reflective fiber-optic hydrophone. In this case a source of probe light, usually from a laser diode is coupled into an optical fiber. The probe light then passes through a fiber-optic component, capable of coupling the probe light into the fiber-optic probe, while coupling the reflected light to another optical fiber, which is guided to a photodetector. The oncoming pressure waves modulate the optical reflectivity of the fiber-optic probe and by measuring the reflected light power with a photodetector, the pressure can be determined. In the simplest form disclosed in WO1989006512A1 a flat and perpendicularly-cleaved (to the light propagation direction) fiber tip is used as a fiber-optic probe and changes of the refraction index of the fluid with the oncoming pressure waves cause the modulation of reflectivity at the fiber-fluid interface.

Different structures can be added to the fiber tip or a shaped (i.e. non-flat) fiber tip can be used, usually to increase sensitivity but possibly lowering the damage threshold and making the probe increasingly more difficult to service in case the probe is damaged by the shockwave.

In order to achieve a fast response of a hydrophone, the probe needs to be as small as possible. In fiber-optic hydrophones, the probe volume is the light beam area times the wavelength of the light used. Usually multimode fibers are used, where sufficient optical power (several watts) can easily be coupled into the fiber, providing sufficient sensitivity of the hydrophone. Such fiber-optic hydrophones have a beam diameter around 100 $\mu$m and wavelength around 1 $\mu$m, already making the probe size significantly smaller when compared to other hydrophone types.

To further increase the bandwidth of a fiber-optic hydrophone and also reduce the lateral position and tilt sensitivity of the probe, a single-mode fiber can be used, with a beam diameter usually around 5 $\mu$m. This, however, results in a high probe light intensity which can have detrimental effects for example by locally heating the fluid around the fiber tip due to the absorption of light in the fluid, possibly leading to boiling and thermally induced damage to the surrounding fluid, which will disrupt the measured signal, while thermally-induced damage is also especially problematic in biological and medical applications of the fiber-optic probe.

Consequently, lower probe-light power needs to be used when using a single-mode fiber, usually in the range of 10 - 200 mW. Using a low power light source and considering the already low reflectivity at the fiber-fluid interface (usually below 0.5%) and low changes of reflectivity with changes of pressure

$$\left( \partial R / \partial p \sim 5 \cdot 10^{-6}\, \mathrm{MPa}^{-1} \right)$$

, where $R$ is the fiber-fluid interface reflection and $p$ is the pressure, poses a challenge for measuring such low-power signals while maintaining a high signal to noise ratio. The technical problem is thus the design of a reflective fiber optic hydrophone that addresses these issues. It is the aim of the invention to provide a fiber-optic hydrophone with a high bandwidth and lowering the lateral position and tilt sensitivity with incident light power < 200 mW to avoid disruption of surrounding fluid.

### State of the art

A basic fiber-optic hydrophone is described in WO1989006512A1, consisting of a fiber-probe with a beam diameter < 100 $\mu$m. The reflected signal is measured with a photodiode with subsequent electronic amplification. Disadvantage of such amplification is lower bandwidth. A possibility to sample a part of the probe-light before reflection is also described to subtract the amplitude fluctuations of the light source from the output signal.

An optical hydrophone with a higher shockwave damage resistance is described in WO2004051203A1. Instead of using the tip of the fiber as a probe, a bulk optically transparent material, being more resistant to shockwave pressure, is used. To maintain the small probe size, which is determined by the light beam size, a set of relay optics is used to focus the light beam onto the bulk-fluid interface. Although this indeed can make a sufficiently small probe area, the bulk material with a much larger size when compared to an optical fiber can disrupt the shockwave propagation in fluid in turn producing measurement that does not reflect the actual pressure in unobstructed shockwave propagation. This solution differs from the present invention in the design of the hydrophone.

A single-mode fiber-optic hydrophone is described in DE19708806C1. Due to the low probe light power of 8 mW, an avalanche photodiode is used to amplify the measured signal. This differs from the present invention. The drawback of using an avalanche photodiode to measure shockwave dynamics is the limited bandwidth of such photodiodes, typically up to around 1 GHz.

Another possible geometry of an optic-fiber hydrophone is described in WO2015030569A1. Here, a laser resonator is built with the fiber-probe tip serving as one of the reflectors forming a resonator. Due to the nature of laser resonators, the output power is highly susceptible to the changes of reflection at the resonator reflector. This provides a very high pressure-sensitivity and such a device is useful as a microphone (i.e. measuring very low pressure waves) in air and using this technique as a hydrophone for measuring pressure in fluids is mentioned as well. However, such a device is limited in response time (bandwidth) by the resonator round-trip time (usually above 10 ns), leading to a bandwidth below 100 MHz and is therefore not suitable as a hydrophone for measuring the shockwave dynamics in fluids, where a bandwidth above 1 GHz is required.

**Description of the solution of the technical problem**

The technical problem is solved as defined in the independent claims, wherein preferred embodiments of the invention are defined in the dependent claims. The main difference between the present invention and known reflective fiber-optic hydrophone is the use of an optical amplifier, which has never been proposed or used before in reflective hydrophones. The main advantage of using fiber-optic amplifiers compared to electronic amplifiers is larger bandwidth, i.e. higher than 1 GHz. This is a consequence of operation of fiber-optic amplifiers, as they amplify the optical signal/power, wherein electronic amplifiers such as avalanche photodiodes amplify the electronic signal created after conversion of light (photons) into electrical current (charge).

The fast and highly sensitive fiber-optic hydrophone according to present invention thus comprises at least:

- A source of probe light coupled into a first single-mode fiber, said source preferably being a laser diode,
- A first fiber-optic element for transmitting at least a part of the incoming probe-light propagating in the forward direction from the first single-mode fiber into a second single-mode fiber and for transmitting at least a part of the reflected light from a probe propagating in the backward direction into a third single-mode fiber,

- the single-mode fiber-optic probe, having a perpendicularly-cleaved or polished single-mode fiber tip, so that the surface of the fiber tip is perpendicular to the probe-light propagation direction, wherein the probe is connected to the second single-mode fiber

- A source of pump light coupled into a fourth optical fiber with a wavelength chosen such that it allows for pumping an optical amplifier, wherein the source of pump light is preferably a laser diode, which is coupled either into a single-mode or multi-mode fiber,

- A second fiber-optic element for combining the reflected probe light from the third single-mode fiber and the pump light from the fourth fiber into a fifth optical fiber, wherein a wavelength-division-multiplexer (WDM) or a multimode pump combiner can be used according to the chosen pump light source,

- a single-mode fiber-optic amplifier for amplifying optical signal from the fifth optical fiber, said amplifier comprising a doped single-mode fiber with length between 20 cm and 5 m to allow for sufficient absorption of the pump light and sufficient amplification of the probe light, wherein the dopant is chosen according to the chosen probe light wavelength, preferably ytterbium-doped or erbium-doped fiber is used, and

- A photodetector with sensitivity above 0.01 A/W and bandwidth above 1 GHz to measure the previously amplified probe light.

The source of the probe light can operate either in continuous-wave (CW) or pulsed-regime, wherein pulse durations are in the range from 10 ns to 1 $\mu$s. The wavelength of the source light is chosen such that the light power can be further amplified in the selected single-mode optical fiber amplifier. Such wavelengths are known to a person skilled in the art of fiber amplifiers. Preferably, the wavelength is chosen around 1 $\mu$m for further amplification in ytterbium-doped fibers or around 1.5 $\mu$m for further amplification in erbium-doped fibers. Other wavelengths can be chosen and the essence of the invention is not limited by the exact wavelength of the probe light. Single mode optic fiber is used with the aim of keeping the sensor as small as possible, so that the beam diameter is approximately 5 um, compared to 50 um or larger in multi-mode fibers. Smaller beam diameter enables higher bandwidth.

Said first fiber-optic element is preferably a fiber-optic circulator, but other elements known to the person skilled in the art of fiber optics can be chosen, such as a fiber-optic coupler.

The amplifier amplifies the probe light power through a mechanism of stimulated light emission and its output is guided to the photodetector that converts the incoming optical power into an electrical signal, which can be measured with for example an oscilloscope.

Additionally, a fiber-optic wavelength filter can be used in order to prevent other wavelengths (i.e. other than the wavelength of the probe light) to enter the fiber optic amplifier and disrupt the measurement. Using the fiber-optic wavelength filter is preferred especially when measuring shockwaves produced by laser-induced breakdown in a fluid as a part of the laser light used to produce the shockwave or light originating from the induced plasma might get coupled into the fiber-optic probe and disrupt the fiber-optic amplifier, producing oscillations and spikes in the measured signal.

Furthermore, a fiber-optic coupler can be used to couple a part of the incoming probe light into another single-mode fiber and using the said part of the probe light to subtract any probe light intensity instabilities (i.e. noise) from the measured signal. This can be achieved for example by guiding both the amplified reflected probe light and the said part of the incoming probe light into a balanced photodiode detector that internally subtracts both signals. In this case the lengths of both inputs to a balanced photodiode detector should be matched such, that the time delay between both signals is significantly less than 1 ns to allow for instabilities suppression in the measured bandwidth (above 1 GHz), preferably the lengths of both fibers should be matched within 1 cm.

The method for measuring with the hydrophone according to invention comprises the following steps: Firstly, the optic probe is placed in the liquid, in which pressure waves should be measured or determined,

Secondly, the source of probe light emits light, which is transmitted to the first single mode fiber and to the fiber optic element such as a fiber optic circulator or coupler and finally through the tip of the single-mode fiber optic probe into the surrounding liquid. A part of the incoming probe light is reflected at the fiber-liquid interface and the amount of reflected power depends on the pressure at the fiber-liquid interface due to the dependence of refractive index of the liquid on the pressure. This dependence can be written as:

$$\frac{\partial n_L}{\partial p} = A,$$

Where $n_L$ is the refractive index of liquid and p is the pressure at the fiber-liquid interface. The value of A is around $1.3 \cdot 10^{-4}$ MPa$^{-1}$ for water. The reflectivity of the fiber-fluid interface is then calculated from Fresnel equations for reflection at the perpendicularly cleaved fiber (i.e. angle of incidence = 0) as:

$$R = \frac{(n_F - n_L)^2}{(n_F + n_L)^2},$$

Where R is the reflectivity of the fiber-liquid interface and $n_F$ and $n_L$ are the refractive indices of fiber (around 1.45 for fused silica glass) and liquid (around 1.32) respectively. From these two equations the changes of reflectivity $(\partial R / \partial p)$ can be obtained. In most cases the changes of fiber refractive index can be neglected due to low compressibility of glass used in typical optical fibers.

Said reflected probe light is guided to the second fiber-optic element, where it is combined with the pump light. This is followed with amplification of the combined reflected probe light in the single-mode fiber-optic amplifier, and measurement of the signal with the photodetector that converts the incoming optical power into an electrical signal, which can be measured with for example an oscilloscope. Measured current/voltage is then conversed into pressure. As the dependency is linear, measured values are multiplied with a known factor dependent on the fluid and refractive index of the glass.

The hydrophone according to the invention may be used in many industrial and medical applications, for example in laser induced breakdown spectroscopy, shock wave lithotripsy and laser vitreolysis.

The fast and highly sensitive reflective fiber-optic hydrophone according to the invention will be described in further detail on the basis of exemplary embodiments and figures, which show:

Figure 1    Schematic view of the fast and highly sensitive fiber-optic hydrophone according to a first embodiment

Figure 2    Schematic view of a second embodiment of the fast and highly sensitive fiber-optic hydrophone allowing for subtraction of the probe light intensity instabilities

Figure 3    Schematic trace of an electronic signal produced by a photodetector when a shockwave is measured by the fast and highly sensitive fiber-optic hydrophone according to the first embodiment

Figure 4    Schematic trace of an electronic signal produced by a photodetector when a shockwave is measured by the second embodiment of the fast and highly sensitive fiber-optic hydrophone

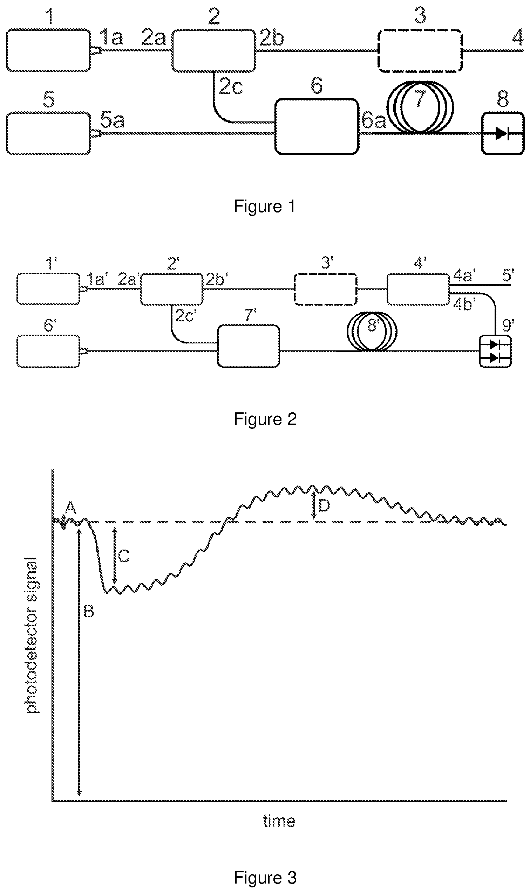

In the scope of the invention as described here and defined in the claims other embodiments of the hydrophone clear to the skilled person in the art of fiber-optic technology are possible, the scope of the invention being solely defined by the appended claims. Figure 1 shows a scheme of the preferred embodiment of the fast and highly sensitive fiber-optic hydrophone and comprises a source of probe light 1 which is coupled into a single-mode fiber 1a. The probe light is guided into a first fiber-optic element 2 that transmits a part of the incoming probe light 2a into a second single-mode fiber 2b and a part of the light reflected from a fiber-optic probe 4 into a third single-mode fiber 2c. The said fiber-optic element 2 can be a fiber coupled circulator or a fiber coupler or any other fiber-optic component meeting the above-mentioned requirements (i.e., transmitting part of the incoming light into the second fiber and transmitting a part of the reflected light into the third fiber), known to a person skilled in fiber-optic technology. The output of the said element 2b is optionally guided into a fiber-optic wavelength filter 3 and finally to a fiber-optic probe 4, which is placed into a fluid and positioned such that the pressure waves to be measured cause sufficient changes to the reflectivity of the probe. The reflected light from the probe coupled into a third fiber 2c is then guided into a second fiber-optic element 6 that combines both the reflected probe light 2c and the pump light from a pump source 5 into a common, fifth fiber 6a. The said element can be either a wavelength-division-multiplexer (WDM) or a multimode pump combiner according to the type of the pump light source 5, which may be a single-mode or a multimode laser diode connected to the second fiber-optic element 6 with a fourth single or multimode optic fiber 5a. The said common fiber guiding both the probe light and the pump light is coupled into a doped fiber amplifier 7, which through a mechanism of stimulated light emission amplifies the probe light power and the output of the doped fiber is guided to a photodetector 8 that converts the incoming optical power into an electrical signal that can be measured with for example an oscilloscope. A typical electrical signal as produced by the photodetector 8 is schematically shown in Figure 3.

Figure 2 shows a second embodiment of the improved fast and highly sensitive fiber-optic hydrophone, which comprises a source of probe light 1' which is coupled into a single-mode fiber 1a'. The probe light is guided into a fiber-optic element 2' that transmits a part of the incoming probe light 2a' into a second single-mode fiber 2b' and a part of the light reflected from fiber-optic probe 5' into a third single-mode fiber 2c'. The said fiber-optic element 2' can be a fiber coupled circulator or a fiber coupler or any other fiber-optic component meeting the above-mentioned requirements, known to a person skilled in fiber-optic technology. The output of the said element 2b' is optionally guided into a fiber-optic wavelength filter 3' and into a fiber-optic coupler 4', which couples a part of the probe light into a fiber-optic probe 5' and another part into a separate single-mode fiber 4b' that is guided into the photodetector to be used to subtract the intensity fluctuations of the probe light source from the measured signal. The reflected light from the probe coupled into a third fiber 2c' is then guided into another fiber-optic element 7' that combines both the reflected probe light 2c' and the pump light from a pump source 6' into a common fiber. The said element can be either a wavelength-division-multiplexer (WDM) or a multimode pump combiner according to the type of the pump light source 6' (single-mode or a multimode laser diode).

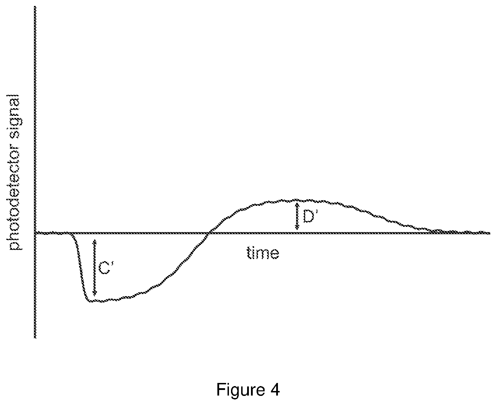

The said common fiber for guiding both the probe light and the pump light is coupled into a doped fiber amplifier 8', which through a mechanism of stimulated light emission amplifies the probe light power and the output of the doped fiber is guided to a balanced photodetector 9' that subtracts the part of the probe light 4b', containing the probe light fluctuations, from the amplified probe light from the amplifier 8' and converts the subtracted optical power into an electrical signal that can be measured with for example an oscilloscope. A typical electrical signal as produced by the photodetector 9' is schematically shown in Figure 4.

In Figure 3 a schematic electrical signal from the photodetector of the first embodiment of the hydrophone is shown. The electrical signal contains the probe light intensity instabilities A and is offset in amplitude B. The offset of the signal results from a non-zero static reflectivity of the fiber-optic probe, when no shockwave is present. A positive pressure peak C and a negative pressure peak D arising from shockwave propagation are shown.

In Figure 4 a schematic electrical signal from the photodetector of the hydrophone according to the second embodiment is shown. The electrical signal contains substantially less probe light intensity modulations and the signal exhibits no offset. Positive pressure peak C' and negative pressure peak D' arising from shockwave propagation are shown.

## Claims

1. A fast and highly sensitive reflective fiber-optic hydrophone for measuring pressure waves in liquids, wherein it comprises at least:

   - a source of probe light (1, 1') coupled into a first single-mode fiber (1a, 1a'),
   - the first single-mode fiber (1a, 1a') coupled into a first fiber-optic element (2, 2') for transmitting at least a part of the incoming probe-light (2a, 2a') propagating in the forward direction from the first single-mode fiber (1a, 1a') into a second single-mode fiber (2b, 2b') and for transmitting at least a part of a reflected light from a probe (4, 5') propagating in the backward direction into a third single-mode fiber (2c, 2c'),

   **characterized in that** the hydrophone further comprises

   - the single-mode fiber-optic probe (4, 5') having a perpendicularly-cleaved or polished single-mode fiber tip, so that the surface of the fiber tip is perpendicular to the probe-light propagation direction, wherein the probe (2, 2') is connected to the second single-mode fiber (2b, 2b'),
   - a source (5, 6') of pump light coupled into a fourth optical fiber (5a) with a wavelength chosen such that it allows for pumping an optical amplifier,
- a second fiber-optic element (6, 7') for combining the reflected probe light from the third single-mode fiber (2c, 2c') and the pump light into a fifth optical fiber (6a),
- a single-mode fiber-optic amplifier (7, 8') for amplifying optical signal from the fifth optical fiber (6a), said amplifier (7, 8') comprising a doped single-mode fiber with length between 20 cm and 5 m to allow for sufficient absorption of the pump light and sufficient amplification of the probe light, wherein the dopant is chosen according to the chosen probe light wavelength, preferably ytterbium-doped or erbium-doped fiber is used, and
- a photodetector (8, 9') with sensitivity above 0.01 A/W and bandwidth above 1 GHz to measure the previously amplified probe light.

2. The fast and highly sensitive reflective fiber-optic hydrophone according to claim 1, wherein said source (1) of probe light is a laser diode.

3. The fast and highly sensitive reflective fiber-optic hydrophone according to claim 1 or 2, wherein said source (1) of the probe light can operate either in continuous-wave (CW) or pulsed-regime, wherein pulse durations are in the range from 10 ns to 1 $\mu$s.

4. The fast and highly sensitive reflective fiber-optic hydrophone according to any claim from 1 to 3, wherein said first fiber-optic element (1a) is a fiber-optic circulator, but other elements known to the person skilled in the art of fiber optics can be chosen, such as a fiber-optic coupler.

5. The fast and highly sensitive reflective fiber-optic hydrophone according to any claim from 1 to 4, wherein said second fiber-optic element (2b) is a wavelength-division-multiplexer (WDM) or a multi-mode pump combiner.

6. The fast and highly sensitive reflective fiber-optic hydrophone according to any claim from 1 to 5, wherein the source (5) of pump light is a laser diode, which is coupled either into the fourth optical fiber, which is either a single-mode or multi-mode fiber.

7. The fast and highly sensitive reflective fiber-optic hydrophone according to any claim from 1 to 6, wherein said amplifier (7) amplifies the probe light power through a mechanism of stimulated light emission and the output of the amplifier (7) is guided to the photodetector (8) that converts the incoming optical power into an electrical signal, which can be measured with for example an oscilloscope.

8. The fast and highly sensitive reflective fiber-optic hydrophone according to any claim from 1 to 7, further comprising a fiber-optic wavelength filter (3, 3') between the first fiber-optic element (1a) and the probe (4).

9. The fast and highly sensitive reflective fiber-optic hydrophone according to any claim from 1 to 8, further comprising a fiber-optic coupler (4') between the between the first fiber-optic element (1a') and the probe (5') or the fiber-optic wavelength filter (3') and the probe (5'), wherein a part of the reflected probe light is guided with a sixth single-mode optic fiber to the photodetector (9'), and wherein the amplified reflected probe light and the said part of the incoming probe light are guided into the photodiode detector that internally subtracts both signals.

10. The fast and highly sensitive reflective fiber-optic hydrophone according to any of the preceding claims for use in industrial and medical applications, for example in laser induced breakdown spectroscopy, shock wave lithotripsy and laser vitreolysis.

11. A method for measuring pressure waves in liquids with the hydrophone according to any of the preceding claims, said method comprising the following steps:

a) the optic probe (4, 5') is placed in the liquid, in which pressure waves should be measured or determined,
b) the source of probe light (1, 1') emits light, which is transmitted to the first single mode fiber (1a, 1a') and to the first fiber optic element (2, 2') and finally through the tip of the single-mode fiber optic probe inserted into the liquid in step a),
c) a part of the incoming probe light from step b) is reflected at the fiber-liquid interface and the amount of reflected power depends on the pressure at the fiber-liquid interface due to the dependence of refractive index of the liquid on the pressure, said reflected probe light is guided to the second fiber-optic element (6, 7'), where it is combined with a pump light emitted by the source (5, 6') of pump light,
d) amplification of the combined reflected probe light from step c) in the single-mode fiber-optic amplifier (7, 8'), and
e) measurement of the signal coming from the output of the amplifier in step d) with the photodetector (8, 9').

**Patentansprüche**

1. Schnelles und hochempfindliches reflektierendes faseroptisches Hydrophon zur Messung von Druckwellen in Flüssigkeiten, das mindestens Folgendes umfasst:

- eine Sondierungslichtquelle (1, 1'), die mit einer ersten Singlemode-Faser (1a, 1a') gekoppelt ist,
- wobei die erste Singlemode-Faser (1a, 1a') mit einem ersten faseroptischen Element (2, 2') gekoppelt ist, um zumindest einen Teil des sich in Vorwärtsrichtung ausbreitenden einfallenden Sondierungslichts (2a, 2a') von der ersten Singlemode-Faser (1a, 1a') in eine zweite Singlemode-Faser (2b, 2b') zu übertragen und um zumindest einen Teil des von einer Sonde (4, 5') reflektierten Lichts, das sich in Rückwärtsrichtung ausbreitet, in eine dritte Singlemode-Faser (2c, 2c') zu übertragen,

**dadurch gekennzeichnet, dass** das Hydrophon ferner umfasst:

- die Singlemode-faseroptische Sonde (4, 5') mit einer senkrecht abgeschnittenen oder polierten Spitze der Singlemode-Faser, sodass die Oberfläche der Faserspitze senkrecht zur Ausbreitungsrichtung des Sondierungslichts steht, wobei die Sonde (2, 2') mit der zweiten Singlemode-Faser (2b, 2b') verbunden ist,
- eine Pumplichtquelle (5, 6'), die mit einer vierten optischen Faser (5a) gekoppelt ist, deren Wellenlänge so gewählt ist, dass sie das Pumpen eines optischen Verstärkers ermöglicht,
- ein zweites faseroptisches Element (6, 7'), um das reflektierte Sondierungslicht aus der dritten Singlemode-Faser (2c, 2c') und das Pumplicht in eine fünfte optische Faser (6a) zu kombinieren,
- einen Singlemode-faseroptischen Verstärker (7, 8') zur Verstärkung des optischen Signals aus der fünften optischen Faser (6a), wobei der Verstärker (7, 8') eine dotierte Singlemode-Faser mit einer Länge zwischen 20 cm und 5 m umfasst, um eine ausreichende Absorption des Pumplichts und eine ausreichende Verstärkung des Sondierungslichts zu ermöglichen, wobei das Dotiermaterial entsprechend der gewählten Wellenlänge des Sondierungslichts ausgewählt wird, vorzugsweise wird eine mit Ytterbium oder Erbium dotierte Faser verwendet, und
- einen Fotodetektor (8, 9') mit einer Empfindlichkeit von über 0,01 A/W und einer Bandbreite von über 1 GHz zur Messung des zuvor verstärkten Sondierungslichts.

2. Das schnelle und hochempfindliche reflektierende faseroptische Hydrophon nach Anspruch 1, wobei die Sondierungslichtquelle (1) eine Laserdiode ist.

3. Das schnelle und hochempfindliche reflektierende faseroptische Hydrophon nach Anspruch 1 oder 2, wobei die Sondierungslichtquelle (1) entweder im Dauerstrichbetrieb (CW) oder im Pulsbetrieb arbeiten kann, wobei die Pulsdauern im Bereich von 10 ns bis 1 $\mu$s liegen.

4. Das schnelle und hochempfindliche reflektierende faseroptische Hydrophon nach einem der Ansprüche 1 bis 3, wobei das erste faseroptische Element (1a) ein faseroptischer Zirkulator ist, es können jedoch auch andere der Fachkraft für Faseroptik bekannte Elemente wie ein faseroptischer Koppler verwendet werden.

5. Das schnelle und hochempfindliche reflektierende faseroptische Hydrophon nach einem der Ansprüche 1 bis 4, wobei das zweite faseroptische Element (2b) ein Wellenlängenmultiplexer (WDM) oder ein Multimode-Pumpkoppler ist.

6. Das schnelle und hochempfindliche reflektierende faseroptische Hydrophon nach einem der Ansprüche 1 bis 5, wobei die Pumplichtquelle (5) eine Laserdiode ist, die entweder mit einer Singlemode- oder Multimode-Faser als vierte optische Faser gekoppelt wird.

7. Das schnelle und hochempfindliche reflektierende faseroptische Hydrophon nach einem der Ansprüche 1 bis 6, wobei der Verstärker (7) die Leistung des Sondierungslichts durch stimulierte Lichtemission verstärkt, und der Ausgang des Verstärkers (7) zum Fotodetektor (8) geführt wird, der die eingehende optische Leistung in ein elektrisches Signal umwandelt, welches beispielsweise mit einem Oszilloskop gemessen werden kann.

8. Das schnelle und hochempfindliche reflektierende faseroptische Hydrophon nach einem der Ansprüche 1 bis 7, ferner umfassend einen faseroptischen Wellenlängenfilter (3, 3') zwischen dem ersten faseroptischen Element (1a) und der Sonde (4).

9. Das schnelle und hochempfindliche reflektierende faseroptische Hydrophon nach einem der Ansprüche 1 bis 8, ferner umfassend einen faseroptischen Koppler (4') zwischen dem ersten faseroptischen Element (1a') und der Sonde (5') oder zwischen dem faseroptischen Wellenlängenfilter (3') und der Sonde (5'), wobei ein Teil des reflektierten Sondierungslichts mit einer sechsten Singlemode-Faser zum Fotodetektor (9') geleitet wird, und wobei das verstärkte reflektierte Sondierungslicht und der genannte Teil des eingehenden Sondierungslichts in einen Fotodiodendetektor geführt werden, der intern beide Signale subtrahiert.

**10.** Das schnelle und hochempfindliche reflektierende faseroptische Hydrophon nach einem der vorhergehenden Ansprüche zur Verwendung in industriellen und medizinischen Anwendungen, beispielsweise in der laserinduzierten Breakdown-Spektroskopie, der Stoßwellenlithotripsie und der Laservitreolyse.

**11.** Verfahren zur Messung von Druckwellen in Flüssigkeiten mit dem Hydrophon nach einem der vorhergehenden Ansprüche, wobei das Verfahren die folgenden Schritte umfasst:

a) a) Die optische Sonde (4, 5') wird in die Flüssigkeit eingebracht, in der die Druckwellen gemessen oder bestimmt werden sollen,
b) b) die Sondierungslichtquelle (1, 1') emittiert Licht, das in die erste Singlemode-Faser (1a, 1a') und das erste faseroptische Element (2, 2') sowie schließlich durch die Spitze der in Schritt a) in die Flüssigkeit eingeführten Singlemode-faseroptischen Sonde übertragen wird,
c) c) ein Teil des in Schritt b) eingekoppelten Sondierungslichts wird an der Faser-Flüssigkeit-Grenzfläche reflektiert, wobei die reflektierte Leistung vom Druck an der Faser-Flüssigkeit-Grenzfläche abhängt, da der Brechungsindex der Flüssigkeit vom Druck abhängt, das reflektierte Sondierungslicht wird zum zweiten faseroptischen Element (6, 7') geleitet, wo es mit dem von der Pumplichtquelle (5, 6') emittierten Pumplicht kombiniert wird,
d) d) Verstärkung des kombinierten reflektierten Sondierungslichts aus Schritt c) im Singlemode-faseroptischen Verstärker (7, 8'), und
e) e) Messung des Signals, das aus dem Ausgang des Verstärkers in Schritt d) kommt, mit dem Fotodetektor (8, 9').

**Revendications**

**1.** Hydrophone à fibre optique par réflexion rapide et hautement sensible pour la mesure des ondes de pression dans les liquides, ledit hydrophone comprenant au moins :

- une source de lumière de sonde (1, 1') couplée dans une première fibre mono-mode (1a, 1a'),
- la première fibre mono-mode (1a, 1a') couplée dans un premier élément à fibre optique (2, 2') pour la transmission d'au moins une partie de la lumière de sonde entrante (2a, 2a') se propageant dans la direction avant depuis la première fibre mono-mode (1a, 1a') dans une deuxième fibre mono-mode (2b, 2b') et pour la transmission d'au moins une partie de la lumière réfléchie provenant d'une sonde (4, 5') et se propageant dans la direction arrière dans une troisième fibre mono-mode (2c, 2c'),

**caractérisé par le fait que** l'hydrophone comprend en outre

- la sonde à fibre optique mono-mode (4, 5') ayant une pointe de fibre mono-mode clivée perpendiculairement ou polie, de façon à ce que la surface de la pointe de fibre soit perpendiculaire à la direction de propagation de la lumière de sonde, la sonde (2, 2') étant connectée à la deuxième fibre mono-mode (2b, 2b'),
- une source (5, 6') de lumière de pompage couplée dans une quatrième fibre optique (5a), ayant une longueur d'onde choisie de façon à ce qu'elle permette le pompage d'un amplificateur optique,
- un second élément à fibre optique (6, 7') destiné à combiner la lumière de sonde réfléchie provenant de la troisième fibre mono-mode (2c, 2c') et la lumière de pompage dans une cinquième fibre optique (6a),
- un amplificateur à fibre optique mono-mode (7, 8') pour l'amplification du signal optique provenant de la cinquième fibre optique (6a), ledit amplificateur (7, 8') comprenant une fibre mono-mode dopée ayant une longueur comprise entre 20 cm et 5 m afin de permettre une absorption suffisante de la lumière de pompage et une amplification suffisante de la lumière de sonde, le dopant étant choisi en fonction de la longueur d'onde de lumière de sonde choisie, de préférence une fibre dopée à l'ytterbium ou à l'erbium est utilisée, et
- un photodétecteur (8, 9') ayant une sensibilité supérieure à 0,01 A/W et une bande passante supérieure à 1 GHz pour mesurer la lumière de sonde préalablement amplifiée.

**2.** Hydrophone à fibre optique par réflexion rapide et hautement sensible selon la revendication 1, ladite source (1) de lumière de sonde étant une diode laser.

**3.** Hydrophone à fibre optique par réflexion rapide et hautement sensible selon les revendications 1 ou 2, ladite source (1) de lumière de sonde pouvant fonctionner soit en onde continue (CW), soit en régime impulsionnel, la durée des impulsions étant comprise entre 10 ns et 1 μs.

**4.** Hydrophone à fibre optique par réflexion rapide et hautement sensible selon l'une quelconque des revendications 1 à 3, où ledit premier élément à fibre optique (1a) est un circulateur optique, mais d'autres éléments connus d'une personne versée dans le domaine de la fibre optique peuvent être choisis, tels qu'un coupleur de fibre optique.

**5.** Hydrophone à fibre optique par réflexion rapide et hautement sensible selon l'une quelconque des revendications 1 à 4, où ledit deuxième élément à fibre optique (2b) est un multiplexeur en longueur d'onde (WDM) ou un combineur de pompe multi-mode.

**6.** Hydrophone à fibre optique par réflexion rapide et hautement sensible selon l'une quelconque des revendications 1 à 5, où la source (5) de la lumière de pompage est une diode laser, qui est couplée à la quatrième fibre optique, qui est soit une fibre mono-mode, soit une fibre multi-mode.

**7.** Hydrophone à fibre optique par réflexion rapide et hautement sensible selon l'une quelconque des revendications 1 à 6, où ledit amplificateur (7) amplifie la puissance de la lumière de sonde au moyen d'un mécanisme d'émission stimulée de lumière et le flux de sortie de l'amplificateur (7) est guidé vers le photodétecteur (8) qui convertit l'énergie optique entrante en signal électrique, qui peut être mesuré avec par exemple un oscilloscope.

**8.** Hydrophone à fibre optique par réflexion rapide et hautement sensible selon l'une quelconque des revendications 1 à 7, comprenant en outre un filtre de longueurs d'onde de fibre optique (3, 3') entre le premier élément à fibre optique (1a) et la sonde (4).

**9.** Hydrophone à fibre optique par réflexion rapide et hautement sensible selon l'une quelconque des revendications 1 à 8, comprenant en outre un coupleur de fibre optique (4') entre le premier élément à fibre optique (1a') et la sonde (5'), ou entre le filtre de longueurs d'onde de fibre optique (3') et la sonde (5'), où une partie de la lumière de sonde réfléchie est guidée par une sixième fibre optique mono-mode vers le photodétecteur (9'), et où la lumière de sonde réfléchie amplifiée et ladite partie de la lumière de sonde entrante sont guidées vers le détecteur à photodiode qui soustrait de façon interne les deux signaux.

**10.** Hydrophone à fibre optique par réflexion rapide et hautement sensible selon l'une quelconque des revendications précédentes pour une utilisation dans des applications industrielles et médicales, par exemple dans la spectroscopie d'émission induite par laser, la lithotritie par ondes de choc et la vitréolyse au laser.

**11.** Méthode de mesure des ondes de pression dans les liquides avec l'hydrophone selon l'une quelconque des revendications précédentes, ladite méthode comprenant les étapes suivantes :

a) la sonde optique (4,5') est placée dans le liquide dans lequel les ondes de pression devraient être mesurées ou déterminées,
b) la source de lumière de sonde (1, 1') émet de la lumière qui est transmise à la première fibre mono-mode (1a, 1a') et au premier élément à fibre optique (2, 2') et finalement à travers la pointe de la sonde à fibre optique mono-mode insérée dans le liquide à l'étape a),
c) une partie de la lumière de sonde entrante de l'étape b) est réfléchie au niveau de l'interface fibre-liquide et la quantité d'énergie réfléchie dépend de la pression au niveau de l'interface fibre-liquide en raison de la dépendance de l'indice de réfraction du liquide par rapport à la pression, ladite lumière de sonde réfléchie étant guidée vers le second élément à fibre optique (6, 7'), où elle est combinée à une lumière de pompage émise par la source (5, 6') de lumière de pompage,
d) l'amplification de la lumière de sonde réfléchie combinée de l'étape c) dans l'amplificateur à fibre optique mono-mode (7, 8'), et
e) la mesure du signal provenant de la sortie de l'amplificateur à l'étape d) avec le photodétecteur (8, 9').

Figure 1

Figure 2

Figure 3

Figure 4

**REFERENCES CITED IN THE DESCRIPTION**

**Patent documents cited in the description**

- WO 1989006512 A1
- WO 2004051203 A1
- DE 19708806 C1
- WO 2015030569 A1